# EUROPEAN PATENT SPECIFICATION

(11) **EP 3 570 849 B1**
(45) Date of publication and mention of the grant of the patent: **25.12.2024**
(21) Application number: 18700426.2
(22) Date of filing: 17.01.2018
(51) Int. Cl.: A61K 33/36, A61K 38/21, A61P 7/00, A61P 7/02, A61P 35/00, A61P 43/00

(54) **PHARMACEUTICAL COMPOSITIONS FOR THE TREATMENT PATIENTS SUFFERING FROM MYELOPROLIFERATIVE DISORDERS**
PHARMAZEUTISCHE ZUSAMMENSETZUNGEN ZUR BEHANDLUNG VON PATIENTEN MIT MYELOPROLIFERATIVEN ERKRANKUNGEN
COMPOSITIONS PHARMACEUTIQUES POUR LE TRAITEMENT DE PATIENTS SOUFFRANT DE TROUBLES MYÉLOPROLIFÉRATIFS

(30) Priority: 18.01.2017 EP 17305053
(43) Date of publication of application: 27.11.2019
(73) Proprietor: INSERM (Institut National de la Santé et de la Recherche Médicale), 75013 Paris (FR); Université Paris Cité, 75006 Paris (FR); Assistance Publique-Hôpitaux de Paris (APHP), 75004 Paris (FR); Université Paris-Sud, 91400 Orsay (FR); Institut Gustave Roussy, 94800 Villejuif (FR); Centre National de la Recherche Scientifique (CNRS), 75016 Paris (FR); College de France, 75005 Paris (FR)
(72) Inventor: DE THE, Hugues, 75010 Paris (FR); CASSINAT, Bruno, 75010 Paris (FR); LALLEMAND-BREITENBACH, Valérie, 75010 Paris (FR); PLO, Isabelle, 94805 Villejuif (FR); VILLEVAL, Jean-Luc, 94805 Villejuif (FR); KILADJIAN, Jean-Jacques, 75010 Paris (FR)
(74) Representative: Inserm Transfert
(86) International application number: PCT/EP2018/051118
(87) International publication number: WO 2018/134260

(56) References cited:
- WO-A1-2016/073825
- US-A1- 2014 286 901
- US-A1- 2016 235 679
- MASLAHL NABIH ET AL: "Specific and Synergistic Targeting of JAK2V617F Cells By Interferon Alpha and Arsenic", BLOOD, AMERICAN SOCIETY OF HEMATOLOGY, US, vol. 132, 29 November 2018 (2018-11-29), pages 52, XP086590529, ISSN: 0006-4971, DOI: 10.1182/BLOOD-2018-99-112518
- EL EIT RABAB ET AL: "Effective targeting of chronic myeloid leukemia initiating activity with the combination of arsenic trioxide and interferon alpha", INTERNATIONAL JOURNAL OF CANCER, vol. 134, no. 4, February 2014 (2014-02-01), pages 988 - 996, XP002773097, DOI: 10.1002/IJC.28427
- VALENT PETER: "Targeting the JAK2-STAT5 pathway in CML.", BLOOD 28 AUG 2014, vol. 124, no. 9, 28 August 2014 (2014-08-28), pages 1386 - 1388, XP002773098, ISSN: 1528-0020
- CANESTRARO M ET AL: "Synergistic antiproliferative effect of arsenic trioxide combined with bortezomib in HL60 cell line and primary blasts from patients affected by myeloproliferative disorders", CANCER GENETICS AND CYTOGENETICS, ELSEVIER SCIENCE PUBLISHING, NEW YORK, NY, US, vol. 199, no. 2, 1 June 2010 (2010-06-01), pages 110 - 120, XP027046479, ISSN: 0165-4608, [retrieved on 20100513]
- BAZARBACHI A ET AL: "Arsenic trioxide and interferon-alpha synergize to induce cell cycle arrest and apoptosis in human T-cell lymphotropic virus type I-transformed cells", BLOOD, THE AMERICAN SOCIETY OF HEMATOLOGY, US, vol. 93, no. 1, 1 January 1999 (1999-01-01), pages 278 - 283, XP002995454, ISSN: 0006-4971

## Description

### FIELD:

The present disclosure is in the field of medicine, in particular haematology.

### BACKGROUND:

Myeloproliferative disorders (MPD) include, among others, polycythemia vera (PV), primary myelofibrosis (PMF), thrombocythemia, essential thrombocythemia (ET), idiopathic myelofibrosis (IMF), chronic myelogenous leukemia (CML), systemic mastocystosis (SM), chronic neutrophilic leukemia (CNL), myelodysplastic syndrome (MDS) and systemic mast cell disease (SMCD). Dysregulation of the JAK2-STAT signalling pathway has been implicated in the development and progression of classical MPD i.e. PV, ET and PMF. Activation of the JAK2/STAT pathway occurs through gain-of-function gene mutations, in particular in the JAK2, MPL, or CALR genes, which represent driver mutations at the origin of the MPD phenotype. The central role of JAK2 in MPD has led to the development of small molecular JAK2 inhibitors that are in clinical use and active in almost all patients with MPD, inducing major effects on splenomegaly, constitutional symptoms related to inflammation and quality of life but minor effects on survival and the MPD clones failing in eradicating stem cells that harbour the mutations. Among other therapies, interferon alpha (IFNα, now used as a pegylated form with prolonged life) is presently the most efficient one targeting the JAK2^{V617F} clone and leading to complete molecular response (CMR). Clinical trials have shown high rates of hematological and molecular responses in PV/ET and in early phase of PMF. However there is still considerable room for improvement. Indeed, about one third of MPN patients do not respond to IFNα and complete molecular response (CMR) is observed in only ≈20% patients. Furthermore, long-term treatments are needed (2-5 years) to obtain CMR, jeopardizing success due to possible long-term toxicity. The reasons for failures, drug resistance and long delays for CMR remain obscure, mostly due to the fact that mechanisms used by IFNα to eradicate the malignant clone are only elusive, thus limiting any improvement of this therapy through combination with synergistic or additive therapeutic molecules.

El Eit, Rabab M., et al. disclose experiments conducted to elucidate the effects of a combination of interferon and arsenic with respect to treating chronic myelogenous leukemia (CML) (El Eit, Rabab M., et al. "Effective targeting of chronic myeloid leukemia initiating activity with the combination of arsenic trioxide and interferon alpha. " International journal of cancer 134.4 (2014): 988-996*).*

WO2016073825 discloses the use of a pegylated type I interferon for treating an infectious disease, cancer, or myeloproliferative disease in a subject in need thereof.

US2016235679 discloses methods of treating malignancies such as tumors or cancers by orally administering lyophilized compositions comprising arsenic to a subject in such need.

### SUMMARY:

The present invention is defined by the claims. In particular the present invention relates to a combination of an interferon polypeptide and an arsenic compound for use in a method of treating a myeloproliferative neoplasm in a patient presenting a dysregulation of the JAK2-STAT signalling pathway wherein the patient harbors the JAK2V617F mutation.

The following detailed description, figures and example do not fall under the scope of the present invention and are present for understanding and illustration purposes only.

Furthermore, any reference in the description to methods of treatment refer to the compounds, pharmaceutical compositions and medicaments of the present invention for use in a method of treatment of the human body by therapy.

### DETAILED DESCRIPTION:

The first object of the present disclosure relates to a method of treating a myeloproliferative disorder in a patient presenting a dysregulation of the JAK2-STAT signalling pathway comprising administering to the patient a therapeutically effective combination of an interferon polypeptide and an arsenic compound.

As used herein, the term "treatment" or "treat" refer to both prophylactic or preventive treatment as well as curative or disease modifying treatment, including treatment of subjects at risk of contracting the disease or suspected to have contracted the disease as well as subjects who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a subject during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a subject during treatment of an illness, e.g., to keep the subject in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., disease manifestation, etc.]).

In particular, the patient suffers from a myeloproliferative neoplasm (MPN). MPNs typically include polycythemia vera (PV), essential thrombocythemia (ET) and primary myelofibrosis (PMF) are a diverse but inter-related group of clonal disorders of pluripotent hematopoietic stem cells that share a range of biological, pathological and clinical features including the relative overproduction of one or more cell types from myeloid origin with growth factor independency/hypersensitivity, marrow hypercellularity, extramedullary hematopoiesis, spleno- and hepatomegaly, and thrombotic and/or hemorrhagic diathesis. An international working group for myeloproliferative neoplasms research and treatment (IWG-MRT) has been established to delineate and define these conditions (see for instance Vannucchi et al, CA Cancer J. Clin., 2009, 59: 171-191), and those disease definitions are to be applied for purposes of this specification.

In particular, the patient suffers from a myelodysplastic syndrome (MDS). MDS is a term used to describe a group of diseases characterized by ineffective hematopoiesis leading to blood cytopenias and hypercellular bone marrow. MDS has traditionally been considered to be synonymous with 'preleukemia' because of the increased risk of transformation into acute myelogenous leukemia (AML).

As used herein the term "JAK2" has its general meaning in the art and refers to the Janus Kinase 2 protein. The amino acid sequence of human JAK2 is well known in the art. Human JAK2 sequences are, for example, represented in the NCBI database (www.ncbi.orgwww.ncbi.nlm.nih.gov/), for example, under accession number NP_004963. Typical MPD associated mutation is the JAK2V617F mutation which refers to the point mutation (1849 G for T) in exon 14, which causes the substitution of phenylalanine for valine at codon 617 in the JAK homology JH2 domain.

The combination therapy of the present disclosure is particularly suitable for eradicating stem cells, which harbour at least one mutation responsible for the dysregulation of the JAK2-STAT signalling pathway and thus efficiently reduces the allele burden of such a mutation (i.e. JAK2V617F).

As used herein, the term "interferon polypeptide" or "IFN polypeptide" is intended to include any polypeptide defined as such in the literature, comprising for example any types of IFNs (type I and type II) and in particular, IFN-alpha, IFN-beta, IFN-omega and IFN-gamma. The term interferon polypeptide, as used herein, is also intended to encompass salts, functional derivatives, variants, muteins, fused proteins, analogs and active fragments thereof. The polypeptide sequences for human interferon-alpha are deposited in database under accession numbers: AAA 52716, AAA 52724, and AAA 52713. The polypeptide sequences for human interferon-beta are deposited in database under accession numbers AAC41702, NP_002167, AAH 96152, AAH 96153, AAH 96150, AAH 96151, AAH 69314, and AAH 36040. The polypeptide sequences for human interferon-gamma are deposited in database under accession numbers AAB 59534, AAM 28885, CAA 44325, AAK 95388, CAA 00226, AAP 20100, AAP 20098, AAK 53058, and NP-000610.

In particular, the interferon is interferon-alpha (IFN-a). The term "IFN-a" encompasses derivatives of IFN-a that are derivatized (e.g., are chemically modified relative to the naturally occurring peptide) to alter certain properties such as serum half-life. As such, the term "IFN-a" includes IFN-a derivatized with polyethylene glycol ("PEGylated IFN-a"), and the like. PEGylated IFN-a, and methods for making same, is discussed in, e.g., U.S. Pat. Nos. 5,382,657; 5,951,974; and 5,981,709. PEGylated IFN-a encompasses conjugates of PEG and any of the above-described IFN-a molecules, including, but not limited to, PEG conjugated to interferon alpha-2a (Roferon, Hoffman La-Roche, Nutley, N.J.), interferon alpha-2b (Intron, Schering-Plough, Madison, N.J.), interferon alpha-2c (Berofor Alpha, Boehringer Ingelheim, Ingelheim, Germany); and consensus interferon as defined by determination of a consensus sequence of naturally occurring interferon alphas (Infergen^{®}, InterMune, Inc., Brisbane, CA.). Thus, in particular, the IFN-a has been modified with one or more polyethylene glycol moieties, i.e., pegylated. Two forms of pegylated-interferon, peginterferon alfa-2a (40 kD) (Pegasys, Hoffmann-La Roche) and peginterferon alfa-2b (12 kD) (Peglntron, Merck), are commercially available, which differ in terms of their pharmacokinetic, viral kinetic, tolerability profiles, and hence, dosing. In particular, Peginterferon alfa-2a (Pegasys) consists of interferon alfa-2a (~20 kD) covalently linked to a 40 kD branched polyethylene glycol (PEG). The PEG moiety is linked at a single site to the interferon alfa moiety via a stable amide bond to lysine. Peginterferon alfa-2a has an approximate molecular weight of 60,000 daltons. The biologic activity of peginterferon- alfa-2a derives from its interferon alfa-2a moiety which impacts both adaptive and innate immune responses against certain viruses. Compared with the native interferon alfa-2a, the peginterferon alfa-2a has sustained absorption, delayed clear. Peginterferon alfa-2a is used as a fixed weekly dose. Peginterferon alfa-2a has a relatively constant absorption after injection and is distributed mostly in the blood and organs.

As used herein, the term "arsenic compound" is intended to include arsenic and any compound having the same biological properties as arsenic. The expression "compound having the same biological properties as arsenic" is understood to mean any compound which, like arsenic, is an inhibitor of phosphatase and/or is capable of creating covalent adducts by binding with dithiol groups. In particular, the arsenic compound is selected from the group consisting of arsenic, arsenic trioxide (As2O3), melarsoprol and arsenic sulfur derivative. Because many of the effects of arsenic are mediated through oxidative stress, agents that like arsenic promote production of reactive oxygen species are encompassed in the disclosure.

As used herein the term "co-administering" as used herein means a process whereby the combination of the interferon polypeptide and the arsenic compound, is administered to the same patient. The interferon polypeptide and the arsenic compound may be administered simultaneously, at essentially the same time, or sequentially. The interferon polypeptide and the arsenic compound need not be administered by means of the same vehicle. The interferon polypeptide and the arsenic compound may be administered one or more times and the number of administrations of each component of the combination may be the same or different. In addition, the interferon polypeptide and the arsenic compound need not be administered at the same site. Typically, the arsenic compounds is preferably administered by the intravenous or oral route, and the interferon polypeptide is administered by the intramuscular or subcutaneous route.

As used the terms "combination" and "combination therapy" are interchangeable and refer to treatments comprising the administration of at least two compounds administered simultaneously, separately or sequentially. As used herein the term "co-administering" as used herein means a process whereby the combination of at least two compounds is administered to the same patient. The at least two compounds may be administered simultaneously, at essentially the same time, or sequentially. The at least two compounds can be administered separately by means of different vehicles or composition. The at least two compounds can also administered in the same vehicle or composition (e.g. pharmaceutical composition). The at least two compounds may be administered one or more times and the number of administrations of each component of the combination may be the same or different.

As used herein, the term "therapeutically effective amount" refers to an amount effective, at dosages and for periods of time necessary, to achieve a desired therapeutic result. A therapeutically effective amount of the active agent may vary according to factors such as the disease state, age, sex, and weight of the individual, and the ability of the active agent to elicit a desired response in the individual. A therapeutically effective amount is also one in which any toxic or detrimental effects of the antibody or antibody portion are outweighed by the therapeutically beneficial effects. The efficient dosages and dosage regimens for the active agent depend on the disease or condition to be treated and may be determined by the persons skilled in the art. A physician having ordinary skill in the art may readily determine and prescribe the effective amount of the pharmaceutical composition required. For example, the physician could start doses of active agent employed in the pharmaceutical composition at levels lower than that required achieving the desired therapeutic effect and gradually increasing the dosage until the desired effect is achieved. In general, a suitable dose of a composition of the present disclosure will be that amount of the compound, which is the lowest dose effective to produce a therapeutic effect according to a particular dosage regimen. Such an effective dose will generally depend upon the factors described above. For example, a therapeutically effective amount for therapeutic use may be measured by its ability to stabilize the progression of disease to decrease the JAK2V617F allele burden or to eradicate the JAK2V617F stem cells. The dose for administration envisaged may be for example from 1 to 50 mg per day, preferably from 10 to 15 mg per day, preferably by the intravenous route, for the arsenic compounds and from 1 to 20 millions of international units (M IU), per day or every two days, preferably from 3 to 9 millions of international units (M IU), per day, for the IFNs.

Typically, the active agent (i.e. the interferon polypeptide or arsenic compound) is administered to the patient in the form of a pharmaceutical composition which comprises a pharmaceutically acceptable carrier. Pharmaceutically acceptable carriers that may be used in these compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene- block polymers, polyethylene glycol and wool fat. For use in administration to a patient, the composition will be formulated for administration to the patient. The compositions of the present disclosure may be administered orally, parenterally, by inhalation spray, topically, rectally, nasally, buccally, vaginally or via an implanted reservoir. The used herein includes subcutaneous, intravenous, intramuscular, intra-articular, intra-synovial, intrasternal, intrathecal, intrahepatic, intralesional and intracranial injection or infusion techniques. Sterile injectable forms of the compositions of this disclosure may be aqueous or an oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono-or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation. The compositions of this disclosure may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include, e.g., lactose. When aqueous suspensions are required for oral use, the active ingredient is combined with emulsifying and suspending agents. If desired, certain sweetening, flavoring or coloring agents may also be added. Alternatively, the compositions of this disclosure may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols. The compositions of this disclosure may also be administered topically, especially when the target of treatment includes areas or organs readily accessible by topical application, including diseases of the eye, the skin, or the lower intestinal tract. Suitable topical formulations are readily prepared for each of these areas or organs. For topical applications, the compositions may be formulated in a suitable ointment containing the active component suspended or dissolved in one or more carriers. Carriers for topical administration of the compounds of this disclosure include, but are not limited to, mineral oil, liquid petrolatum, white petrolatum, propylene glycol, polyoxyethylene, polyoxypropylene compound, emulsifying wax and water. Alternatively, the compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water. Topical application for the lower intestinal tract can be effected in a rectal suppository formulation (see above) or in a suitable enema formulation. Patches may also be used. The compositions of this disclosure may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

The disclosure will be further illustrated by the following figures and examples. However, these examples and figures should not be interpreted in any way as limiting the scope of the present disclosure.

### FIGURES:

All figures refer to animals transplanted with a mixture of JAK2-mutant and wild type bone marrow cells to mimic the human disease
Figure 1 shows the white blood cells and platelets levels in mice treated with IFN and Arsenic or their combination.
Figure 2 shows the haematocrit and hemoglobin levels in mice treated with IFN and Arsenic or the combination.
Figure 3 shows the red blood cells number and volume levels in mice treated with IFN and Arsenic or the combination.
Figure 4 shows the JAK2V617F allele burden in granulocytes and platelets in mice treated with IFN and Arsenic or the combination
Figure 5 shows the JAK2V617F "allele burden" (GFP) in red blood cells and B lymphocytes in mice treated with IFN and Arsenic or the combination
Figure 6 shows the organ cellularity in mice treated with IFN and Arsenic or the combination.
Figure 7 shows that a selective and synergistic impairment of the growth of mutant erythroid colonies is observed with the combination of IFN and Arsenic in clonogenic methyl-cellulose cultures of patient progenitors.

### EXAMPLE 1:

### Methods:

The MPN mouse model (JAK2^{V617F} KI /vav-Cre) and the preclinical effect of IFNa alone in this model were previously described (Hasan et al. JAK2V617F expression in mice amplifies early hematopoietic cells and gives them a competitive advantage that is hampered by IFNα. Blood 2013). Briefly, this model mimics human polycythemia vera (PV) transforming into secondary myelofibrosis after 5 months. A 12-13 week IFNa treatment prevented disease development and eradicated disease-initiating cells (secondary recipient assay) by specifically targeting JAK2^{V617F} cells at an early stage of differentiation. The preclinical model is generated in normal mice by transplanting a mixture of 60% WT and 40% KI JAK2^{V617F} bone marrow (BM) cells. The KI JAK2^{V617F} mice are previously crossed with Ubi-GFP transgenic mice in order to marked the JAK2^{V617F} cells and follow the JAK2^{V617F} allele burden in blood and tissues by FACS analysis using GFP. Analysis comprised: i) Blood cell parameters and allele burden (GFP) / 2 weeks, ii) BM / SP analysis (CFC, allele burden in sorted LSK/SLAM, histology, iii) Evaluation of disease-initiating cells in secondary recipients and iv) Evaluation of cure or relapse in treated mice after cessation of treatment.

### Results:

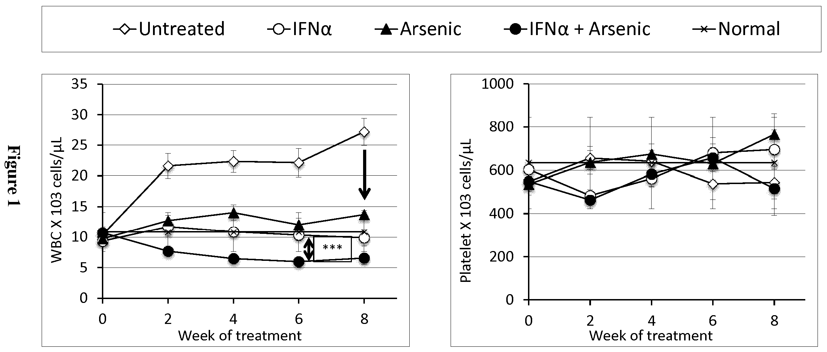

IFN alone or IFN + ARS significantly decreased (p<0.05) the leucocytosis to normal values (Figure 1). The combination significantly (p<0.05) increased the effect of ARS alone or IFN alone (Figure 1). No effect of ARS alone, IFN alone or IFN + ARS on platelet counts in this experiment (Figure 1), in contrast to a previous experiment where IFN alone induced a thrombopenia that was slightly improved by the IFN + ARS combination at week 6 and 8 of treatment (p<0.05, data not shown).

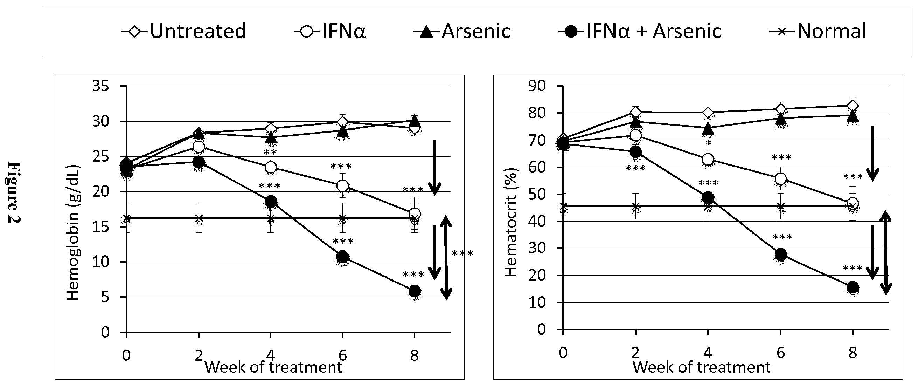

We observed no effect of ARS alone on polycythemia measured on hemoglobin or hematocrit levels (Figure 2). IFN or IFN + ARS decreased the polycythemia to normal levels with IFN (P<0001) and to severe anemia with the combination (P<0,0001) (Figure 2). ARS potentiated efficiently the effect of IFN on hemoglobin or hematocrit level reduction.

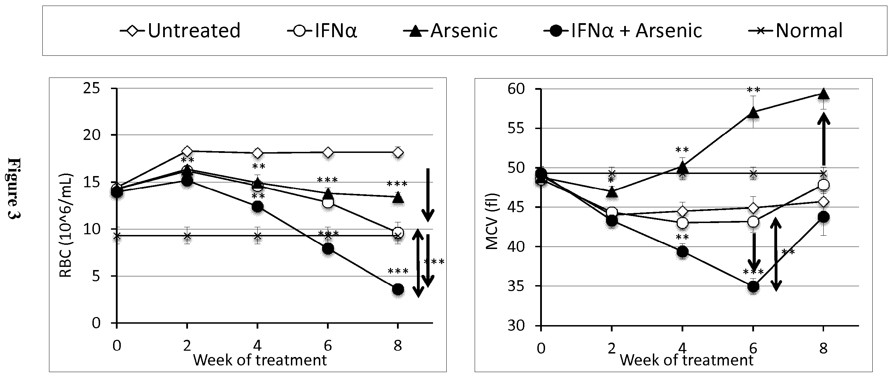

ARS, IFN and IFN + ARS decreased the red blood cell levels (P<0,0001). ARS efficiently potentiated the effect of IFN on the decrease in RBC levels (Figure 3). ARS increase (p<0.005) the RBC volume to macrocytosis (explaining the unchanged levels of HB and Ht despite the reduction in RBC concentration) (Figure 3). IFN has no effect of MCV and the combination only transiently decrease the MCV (p<0.005) (Figure 3).

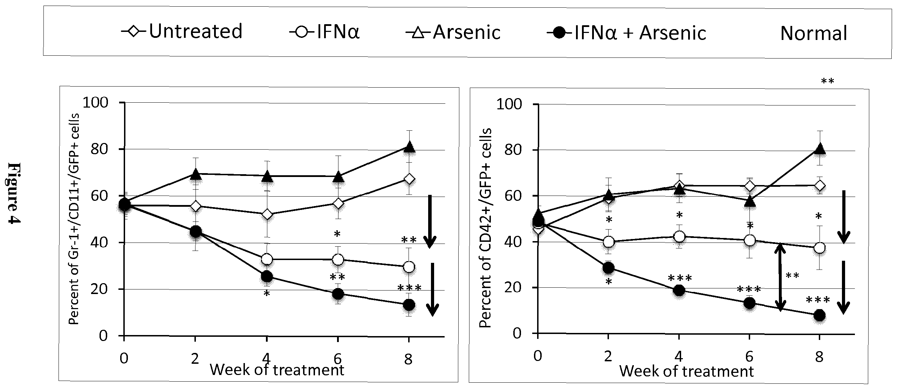

We observed no effect of ARS alone but a tendency to increase (≈20% of NT) the JAK2V617F allele burden in WBC (Figure 4). IFN decreases the WBC and platelet (≈ 40% of NT) allele burdens (p<0.05) (Figure 4) as previously described (Hasan et al. Blood 2013). The combination IFN + ARS improves the effect of IFN alone on the WBC (80% of NT) and platelet (85% of NT) JAK2V617F allele burdens (p<0.001) (Figure 4). The effect on platelet is significantly more elevated than with IFN alone.

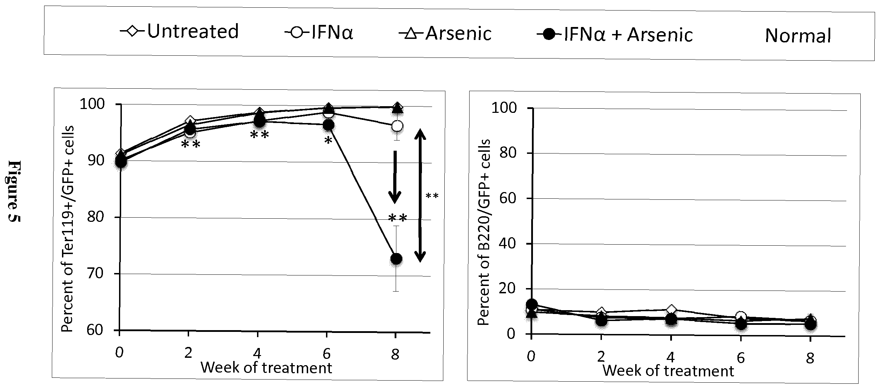

We observed no effect of ARS or IFN on RBC from neoplastic origin (JAK2V617F+) (Figure 5). We observed a decrease in RBC allele burden from neoplastic origin (JAK2V617F+) with the combination IFN + ARS (p<0.05) (Figure 5). The effect is significantly more elevated than with IFN alone after 8 weeks of treatment.

We observed no effect on the low B lymphocyte allele burden (Figure 5) and a minor increase in the low T cell allele burden (from 3 to 5%) after IFN or IFN+ARS (p<0.05) (data not shown).

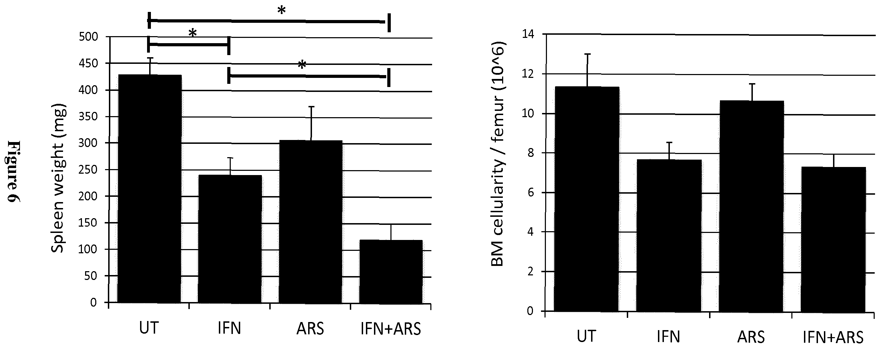

We observed a decrease of splenomegaly after IFN and the combination IFN+ARS significantly reduced splenomegaly compared to IFN alone (p<0.05) to a normal spleen weight (Figure 6). There was a tendency to a decrease in BM cellularity after IFN or IFN+ARS (Figure 6).

ARS increased the allele burden in SLAM cells (p<0.05) and IFN decreased the allele burden in all early cells examined (p<0.05) (data not shown). The combination IFN+ARS improved the effect of IFN alone on allele burden, especially in SLAM (p<0.005) (data not shown). ARS alone increased the allele burden in spleen progenitor cells (p<0.05) (data not shown). The combination IFN+ARS but not IFN alone decreased the allele burden in BM (data not shown) and SP (p<0.05) progenitor cells (data not shown).

ARS or IFN did not modify the total number of myeloid progenitor cells. The combination IFN+ARS tend to decrease the total number of progenitors (p=0.05) (data not shown).

Bone marrow cells from treated animals were transplanted into secondary irradiated recipients to evaluate the effect of treatment on disease-initiating cells. All animals transplanted with the bone marrow from non-treated or ARS-treated animals developed 5 weeks after transplantation the disease with high WBC and Ht. Two third (4/6) of animals transplanted with the bone marrow from IFN-treated animals developed also the disease. In contrast, none of the animals transplanted with the bone marrow from IFN+ARS-treated animals developed the disease (data not shown). Similarly, no or a very low JAK2V617F allele burden was found in WBC, platelet and red blood cells from animals transplanted with the bone marrow from IFN+ARS-treated animals in contrast to the other groups (untreated, ARS or IFN-treated animals) (data not shown).

Some animals were examined after the end of treatment. All the non-treated or ARS-treated animals relapse after cessation of treatment. In contrast, 5/7 (≈ 70%) animals treated with IFN relapsed and 3/7 (≈ 40%) animals treated with IFN + ARS relapsed 10 or 4 weeks after cessation of treatment. For example in experience 1, 10 weeks after cessation of treatment, 2 animals out of 4 treated with IFN + ARS displayed at the same time normal WBC counts, hematocrits and spleen weights in contrast to animal treated with IFN or untreated (data not shown).

### Conclusions:

IFN alone:
   - decreased leucocytosis to normal WBC count levels
   - decreased platelet levels (thrombopenia) (excepted in one experiment but that was unusual (1 out of 5)
   - Decreased Ht, Hb and RBC levels (p<0.001) and prevents microcytosis (increase RBC volume)
   - decreased of splenomegaly
   - decreased the allele burdens in WBC, platelet, RBC and all early cells
   - partially suppressed disease initiating cells and prevents relapse after treatment cessation
ARS alone:
   - significantly decreased (p<0.05) the leucocytosis
   - had no effect on platelet counts
   - had no effect on Hb or Ht levels but decreased RBC levels and increased RBC volume (p<0.005)
   - had no effect on WBC allele burden (tendency to increase it)
   - increased the allele burden in early cells.
The IFN + ARS combination:
   - improves the decrease in WBC induced by ARS or IFN alone
   - has no effect on platelet count or improves the thrombocytopenia induced by IFN alone
   - potentiates the decreased in hematocrit, hemoglobin and RBC levels induced by IFN alone and caused a transient microcytosis (decrease in RBC volume, MCV)
   - decreased splenomegaly to normal weight, improving the IFN alone effect.
   - improves IFN effect on allele burden decreases in WBC, platelet, RBC and early cells
   - improved IFN effect on eradication of disease-initiating cells and prevention of relapse after treatment cessation

These studies demonstrate that combining ARS with IFN improves most of the benefits provided by IFN alone during MPN treatment. This was evidenced on:
- Correction of polycythemia
- Correction of leukocytosis
- Correction of thrombocytopenia induced by IFN
- Reduction of circulating granulocytes, platelet and red blood cells derived from the neoplastic JAK2V617F clones
- Reduction of bone marrow and spleen neoplastic JAK2V617F progenitor and stem cells
- Suppression of JAK2V617F-positive disease initiating cells
- Prevention of disease relapse after treatment cessation

Overall, this study strongly suggests that MPN patients will strongly benefit from a treatment combining ARS with IFN. This treatment should improve the percentage of completed molecular remission induced by IFN but may be associated with anemia that will need to be monitored and prevented during the treatment before sign of remission.

These studies suggest that the mechanism of action of IFN therapy in MPN patients may be associated with common pathways used by IFN and arsenic. Preliminary results have shown that the level of PML, a common target of IFN and arsenic, is decreased in murine JAK2V617F bone marrow and spleen cells and increase PML level and association to nuclear bodies may be implicated in the eradication of MPN JAK2V617F stem cells.

### EXAMPLE 2:

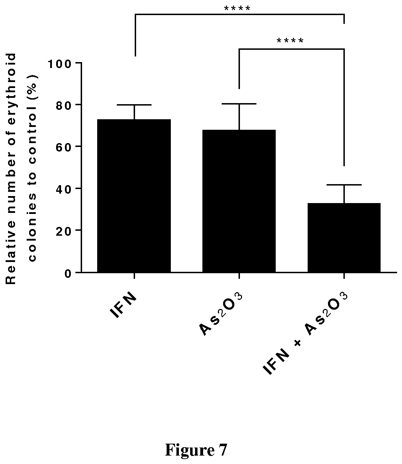

Exploring combination of IFN and ARS in clonogenic methyl-cellulose cultures of patient progenitors, a selective and synergistic impairment of the growth of mutant erythroid colonies was observed (Figure 7), arguing for the relevance of the mouse observations to primary human cells.

### REFERENCES:

Throughout this application, various references describe the state of the art to which this disclosure pertains.

## Claims

1. A combination of an interferon polypeptide and an arsenic compound for use in a method of treating a myeloproliferative neoplasm in a patient presenting a dysregulation of the JAK2-STAT signalling pathway wherein the patient harbors the JAK2V617F mutation.

2. The combination of an interferon polypeptide and an arsenic compound for use according to claim 1 wherein the patient suffers from a myeloproliferative neoplasm (MPN)selected from the group consisting of polycythemia vera (PV), essential thrombocythemia (ET) and primary myelofibrosis (PMF).

3. The combination of an interferon polypeptide and an arsenic compound for use according to claim 1 wherein the interferon polypeptide is an interferon-alpha (IFN-a) polypeptide.

4. The combination of an interferon polypeptide and an arsenic compound for use according to claim 1 wherein the interferon-alpha is pegylated.

5. The combination of an interferon polypeptide and an arsenic compound for use according to claim 1 wherein the arsenic compound is selected from the group consisting of arsenic, arsenic trioxide (As2O3), melarsoprol and arsenic sulfur derivative.

## Patentansprüche

1. Kombination aus einem Interferon-Polypeptid und einer Arsenverbindung zur Verwendung in einem Verfahren zur Behandlung eines myeloproliferativen Neoplasmas bei einem Patienten mit einer Dysregulation des JAK2-STAT-Signalwegs, wobei der Patient die JAK2V617F-Mutation aufweist.

2. Kombination aus einem Interferon-Polypeptid und einer Arsenverbindung zur Verwendung nach Anspruch 1, wobei der Patient an einem myeloproliferativen Neoplasma (MPN) leidet, das aus der Gruppe bestehend aus Polycythemia vera (PV), essentieller Thrombozythämie (ET) und primärer Myelofibrose (PMF) ausgewählt ist.

3. Kombination aus einem Interferon-Polypeptid und einer Arsenverbindung zur Verwendung nach Anspruch 1, wobei das Interferon-Polypeptid ein Interferon-alpha (IFN-a) Polypeptid ist.

4. Kombination aus einem Interferon-Polypeptid und einer Arsenverbindung zur Verwendung nach Anspruch 1, wobei das Interferon-alpha pegyliert ist.

5. Kombination aus einem Interferon-Polypeptid und einer Arsenverbindung zur Verwendung nach Anspruch 1, wobei die Arsenverbindung aus der Gruppe bestehend aus Arsen, Arsentrioxid (As2O3), Melarsoprol und Arsenschwefelderivat ausgewählt ist.

## Revendications

1. Combinaison d'un polypeptide d'interféron et d'un composé d'arsenic destinée à être utilisée dans un procédé de traitement d'un néoplasme myéloprolifératif chez un patient présentant un dérèglement de la voie de signalisation JAK2-STAT dans laquelle le patient est porteur de la mutation JAK2V617F.

2. Combinaison d'un polypeptide d'interféron et d'un composé d'arsenic destinée à être utilisée selon la revendication 1, dans laquelle le patient souffre d'un néoplasme myéloprolifératif (MPN) choisi dans le groupe consistant en la polycythémie vraie (PV), la thrombocytémie essentielle (ET) et la myélofibrose primitive (PMF).

3. Combinaison d'un polypeptide d'interféron et d'un composé d'arsenic destinée à être utilisée selon la revendication 1, dans laquelle le polypeptide d'interféron est un polypeptide d'interféron alpha (IFN-a).

4. Combinaison d'un polypeptide d'interféron et d'un composé d'arsenic destinée à être utilisée selon la revendication 1, dans laquelle l'interféron alpha est pégylé.

5. Combinaison d'un polypeptide d'interféron et d'un composé d'arsenic destinée à être utilisée selon la revendication 1, dans laquelle le composé d'arsenic est choisi dans le groupe consistant en l'arsenic, le trioxyde d'arsenic (As2O3), le mélarsoprol et un dérivé soufré d'arsenic.
